(19) Europäisches Patentamt
European Patent Office
Office européen des brevets

(11) **EP 1 170 274 A1**

(12) **EUROPÄISCHE PATENTANMELDUNG**

(43) Veröffentlichungstag:
**09.01.2002 Patentblatt 2002/02**

(51) Int Cl.[7]: **C07C 29/00**, C07C 29/50, C07C 51/367

(21) Anmeldenummer: **00113103.6**

(22) Anmeldetag: **28.06.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **KAJO-Chemie, chemische und mineraloelhaltige Produkte GmbH**
**59609 Anröchte (DE)**

(72) Erfinder: **Lewen, Bernd, Dr.**
**59555 Lippstadt (DE)**

(74) Vertreter: **Best, Michael, Dr. et al**
**Lederer, Keller & Riederer Patentanwälte**
**Prinzregentenstrasse 16**
**80538 München (DE)**

(54) **Verfahren zur Herstellung von Polyolen**

(57) Die vorliegende Erfindung betrifft ein Verfahren zur Herstellung von Polyolen aus nativen Ölen und mit diesem Verfahren erhältliche Polyole. Diese Polyole eignen sich insbesondere als Monomerbausteine zur Herstellung von Kunststoffen und insbesondere von Polyurethanen.

EP 1 170 274 A1

## Beschreibung

Die vorliegende Erfindung betrifft ein Verfahren zur Herstellung von Polyolen aus nativen Ölen, mit diesem Verfahren erhältliche Polyole und deren Verwendung zur Herstellung von Kunststoffen.

Polyole, d.h. polyfunktionelle Alkohole mit mindestens zwei alkoholischen Hydroxygruppen pro Molekül, werden als Monomerbausteine zur Herstellung von Kunststoffen und insbesondere von Polyurethanen eingesetzt. Polyurethane werden beispielsweise durch Umsetzung von Polyolen mit di- oder polyfunktionellen Isocyanaten erhalten. Als Beispiele für geeignete Polyole sind Glycole, Glycerin, Sorbit, Inosit und Pentaerythrit zu nennen.

In der Polymerchemie sind darüber hinaus die Polyalkylenglycole als monomere Polyolbausteine wichtig. Hierbei handelt es sich um höher molekulare Glycole wie Polyethylenglycol, Polyether- und Polyesterpolyole.

Polyole stellen eine umfangreiche Gruppe von Polyurethanrohstoffen dar. Entscheidend für deren Auswahl sind die geforderten Eigenschaften des Polyurethans. In erster Linie kommen Polyole mit 2-3 Hydroxygruppen zum Einsatz, für spezielle Zwecke, z.B. als Vernetzer, werden aber auch Polyole mit höheren Funktionalitäten verwendet.

Zur Herstellung von Kunststoffen mit neuartigen Eigenschaften ist es wünschenswert, neuartige Monomerbausteine für die Kunststoffsynthese zur Verfügung zu stellen. Darüber hinaus ist es auch aus Gründen des Umweltschutzes wünschenswert, Monomere für die Kunststoffindustrie zur Verfügung zu stellen, die nicht wie herkömmlich zumeist üblich aus Erdöl gewonnen werden, sondern aus nachwachsenden Rohstoffen.

P. Daute et al. schlagen in Fat Sci. Technol. 95, 91-4 (1993) vor, ausgehend von ungesättigten Fettstoffen unter Nutzung der für Fettalkohole etablierten Hydrier-Technologie polyfunktionelle Alkohole zu gewinnen. Insbesondere wird vorgeschlagen, ungesättigte Fettalkohole, beispielsweise mittels Persäuren zu epoxidieren und anschließend durch Ringöffnung mit Mono- oder Polyalkoholen zu fettchemisch basierten Polyolen umzusetzen. Darüber hinaus wird vorgeschlagen, ungesättigte Fettalkohole zu dimerisieren, um Dimeralkohole zu erhalten.

Nachteilig an diesen Verfahren ist, daß die als Edukte eingesetzten nativen Öle und Fette zunächst durch Esterspaltung und Reduktion in die Fettalkohole überführt werden müssen. Darüber hinaus ist eine Epoxidierung mit Persäuren in technischem Maßstab riskant, da es sich um explosive Verbindungen handelt.

In der Lack- und Firnisindustrie ist darüber hinaus die Verwendung sogenannter trockener Öle bekannt. Trokkene Öle weisen eine Iodzahl (IZ) > 170 auf und zeichnen sich dadurch aus, daß sie in Gegenwart von Luftsauerstoff durch Polymerisation verfestigen. Diese Eigenschaft wird beim Aushärten von Lacken und Firnissen genutzt.

Neben nativen Ölen werden auch chemisch modifizierte trockene Öle eingesetzt, wobei neben verschiedenen anderen Modifikationsmöglichkeiten das Verblasen des Öls mit Luft zur Erhöhung der Viskosität des Öls bekannt ist (Ullmann's Encyclopedia of Industrial Chemistry, 5. Aufl., Bd. A9, Seiten 55-68).

Problematisch beim Verblasen von Ölen ist, daß sich zwar zunächst über einen Autoxidationsschritt Hydroperoxide bilden, diese jedoch in einem zweiten Schritt leicht zu Alkoxy- und Hydroxylradikalen zerfallen, die dann in einer Radikalkettenreaktion zu einer Polymerisation und damit Verfestigung des Öls führen (Ullmann's, a.a.O., Seite 63).

Eine Aufgabe der vorliegenden Erfindung besteht darin, neuartige Polyole als Monomerbausteine für die Kunststoffherstellung zur Verfügung zu stellen. Eine weitere Aufgabe der vorliegenden Erfindung besteht darin, ein einfaches Verfahren zur Herstellung der Polyole zur Verfügung zu stellen.

Es wurde nun gefunden, daß durch ein Verblasen von nativen Ölen mit einem sauerstoffhaltigen Gas dann für die Kunststoffherstellung geeignete Polyole erhalten werden können, wenn das native Öl unter den gegebenen Reaktionsbedingungen für einen bestimmten Zeitraum verblasen wird.

Die vorliegende Erfindung betrifft somit ein Verfahren zur Herstellung von Polyolen, dadurch gekennzeichnet, daß ein natives Öl solange mit einem sauerstoffhaltigen Gas umgesetzt wird, bis das erhaltene Produkt eine kinematische Viskosität bei 40°C im Bereich von 100-8000 $mm^2/s$ und eine mittlere Anzahl an Hydroxygruppen pro Molekül von mindestens zwei aufweist.

Unter Polyolen werden vorliegend polyfunktionelle Alkohole mit mindestens zwei alkoholischen Hydroxygruppen pro Molekül verstanden. Hierbei ist jedoch zu berücksichtigen, daß in dem erfindungsgemäßen Verfahren eine Mischung aus verschiedenen modifizierten Fettmolekülen und deren Oligomeren erhalten wird. Auch wenn angestrebt wird, daß möglichst alle der erhaltenen Moleküle mindestens zwei Hydroxygruppen aufweisen, so wird vorliegend unter Polyol auch eine Mischung von Molekülen verstanden, die eine mittlere Anzahl an Hydroxygruppen pro Molekül von mindestens zwei aufweisen. Bevorzugt werden Polyole mit einer mittleren Anzahl an Hydroxygruppen pro Molekül von 2-3, wobei je nach angestrebtem Verwendungszweck jedoch auch Polyole mit einer größeren mittleren Anzahl an Hydroxygruppen erhalten werden können.

Die mittlere Anzahl der Hydroxygruppen pro Molekül wird im folgenden auch als Funktionalität des Polyols bezeichnet.

Für das erfindungsgemäße Verfahren werden als Edukt native Öle eingesetzt. Hierbei kann es sich sowohl um pflanzliche als auch um tierische Öle handeln, wobei pflanzliche Öle als nachwachsende Rohstoffe bevorzugt sind. Es können auch Mischungen aus zwei oder mehreren nativen Ölen eingesetzt werden.

Die Öle sollten ungesättigte, vorzugsweise mehrfach ungesättigte Fettsäurereste umfassen, da die Autoxidationsreaktion mit Sauerstoff an ungesättigten Fettsäureresten leichter abläuft. Besonders geeignet sind Öle, die Fettsäurereste mit konjugierten Doppelbindungen umfassen, da die bei der Autoxidation zunächst gebildeten Radikale durch konjugierte Doppelbindungen stabilisiert werden.

In einer besonders bevorzugten Ausführungsform weist das native Öl einen Mindestgehalt an ungesättigten Fettsäureresten von ca. 80% bezogen auf die Gesamtzahl der Fettsäurereste auf. Außerdem werden für die vorliegende Erfindung sogenannte halbtrockene Öle mit einer Iodzahl (IZ) von 100-170 bevorzugt.

Beispielsweise kann für das vorliegende Verfahren Calendulaöl, Rapsöl oder Sojaöl eingesetzt werden. Calendulaöl wird aus Ringelblumen (Calendula Officinalis) gewonnen, Rapsöl aus Raps (Brasica Napus).

Calendulaöl, das für das vorliegende Verfahren besonders bevorzugt eingesetzt wird, umfaßt als wesentlichen Fettsäurerest zu 58% Calendicsäure, eine C18 Säure mit drei konjugierten Doppelbindungen. Als weitere Säurereste umfaßt Calendulaöl 29% Linolsäure, 4% Ölsäure, 3% Palmitinsäure, 1% Stearinsäure und 1% Linolensäure. Rapsöl umfaßt als wesentliche Fettsäurereste 60% Ölsäure, 20% Linolsäure, 8% Linolensäure, 4% Palmitinsäure und 2% Stearinsäure.

Die Pflanzenöle werden durch Pressen oder Extraktion gewonnen und enthalten üblicherweise zunächst verschiedene Begleitstoffe, die die weitere Verarbeitung der Öle beeinträchtigen können. Daher werden erfindungsgemäß bevorzugt raffinierte Öle eingesetzt. Der Raffinationsgrad kann dabei dem von Speiseölen entsprechen, so daß sich ein reines und klares Öl mit mildem Geruch und ausreichender Lagerstabilität ergibt. Durch die Raffination wird zusätzlich eine weitgehend gleichbleibende Qualität des Edukts garantiert.

Als zweites Edukt wird in dem erfindungsgemäßen Verfahren ein sauerstoffhaltiges Gas eingesetzt. Hierbei kann es sich sowohl um reinen Sauerstoff als auch um Sauerstoff in Mischung mit anderen, bei der vorliegenden Reaktion inerten Gasen handeln. Aus Kostengründen wird bevorzugt Luft als sauerstoffhaltiges Gas verwendet.

Das eingesetzte sauerstoffhaltige Gas sollte vor seiner Verwendung getrocknet werden, um ein Einschleppen von Wasser in die Reaktionsmischung und damit in die hergestellten Polyole zu verhindern. Wasser kann nicht nur zu unerwünschten Nebenreaktionen bei der Autoxidation des nativen Öls führen, sondern auch bei der späteren Kunststoffherstellung beispielsweise durch Reaktion mit zugesetzten Isocyanatmonomeren störend wirken.

Zur Umsetzung wird das sauerstoffhaltige Gas in das native Öl eingeblasen. Vorteilhaft wird das Gas fein verteilt beispielsweise durch eine Fritte unterhalb des Flüssigkeitsspiegels eingeblasen, um eine gleichmäßige und vollständige Reaktion zu gewährleisten. Zur besseren Durchmischung kann das Öl beispielsweise zusätzlich gerührt werden.

Ohne an eine Theorie gebunden sein zu wollen wird angenommen, daß der in dem eingeblasenen Gas enthaltene Sauerstoff in einer radikalischen Reaktion mit den Fettsäureresten des Öls unter Bildung von Hydroperoxiden bzw. Peroxidradikalen als Zwischenprodukt reagiert. Ein angenommener Mechanismus der Radikalkettenreaktion ist nachfolgend dargestellt:

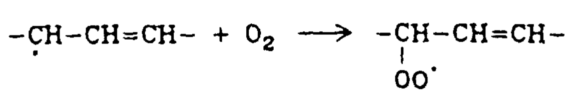

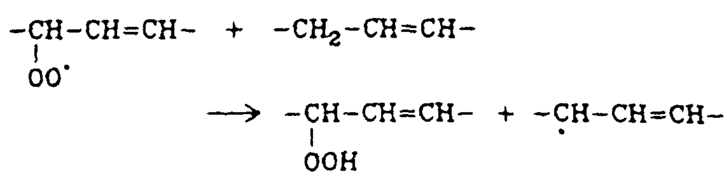

Die in dem ersten Reaktionsschritt gebildeten Hydroperoxide sind instabil und zerfallen leicht in freie Alkoxy- und Hydroxylradikale:

$$R\text{-}OOH \rightarrow RO^{\bullet} + {}^{\bullet}OH$$

Diese Radikale können auf verschiedenen Wegen weiter reagieren, wobei Hydroxgruppen entstehen können oder es zu einer Polymerisation kommen kann. Eine Polymerisation könnte beispielsweise über folgende Reaktion ausgelöst werden.

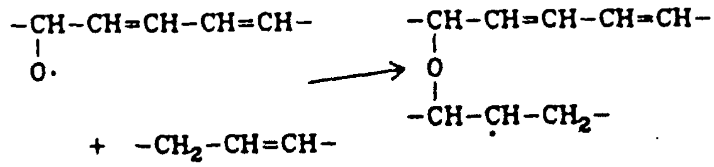

Aus den vorstehenden Reaktionsabläufen wird deutlich, daß bei der Steuerung des erfidungsgemäßen Verfahrens darauf zu achten ist, daß nicht nur eine unkontrollierte Polymerisation abläuft, die zu einem festen, unbrauchbaren Produkt führen würde, sondern daß ein für die Weiterverarbeitung zu Kunststoffen brauchbares Produkt erhalten wird, das keinen zu hohen Polymerisationsgrad aufweist und in dem insbesondere jedes Molekül noch genügend Hydroxygruppen umfaßt, damit diese bei der späteren Kunststoffherstellung eine Polymerisation ermöglichen.

Es wurde gefunden, daß dieses Ziel dadurch erreicht werden kann, daß die Umsetzung mit dem sauerstoffhaltigen Gas nur so lange durchgeführt wird, bis das erhaltene Produkt eine kinematische Viskosität bei 40°C im Bereich von 100-8000 $mm^2/s$ aufweist. Bevorzugt hat das Produkt eine kinematische Viskosität bei 40°C im Bereich von 400-5000 $mm^2/s$ und besonders bevorzugt im Bereich von 1000-3000 $mm^2/s$. Außerdem darf die Umsetzung erst dann abgebrochen werden, wenn das Produkt eine mittlere Anzahl an Hydroxygruppen pro Molekül von mindestens zwei aufweist.

Durch diese Rahmenbedingungen kann der Fachmann in Abhängigkeit von dem eingesetzten Öl geeignete Reaktionsbedingungen wählen. Durch Messung der kinematischen Viskosität sowie der Bestimmung der mittleren Anzahl an Hydroxygruppen pro Molekül in dem Reaktionsprodukt kann der Fachmann die Zeitdauer bestimmen, während der das Öl mit dem sauerstoffhaltigen Gas umgesetzt werden muß. Erfindungsgemäß muß die Reaktion bei vorgegebener Temperatur so lange durchgeführt werden, bis das Produkt eine kinematische Viskosität bei 40°C im Bereich von 100-8000 $mm^2/s$ und eine mittlere Anzahl an Hydroxygruppen pro Molekül von mindestens zwei aufweist. Sollte bei einer vorgegebenen Temperatur beispielsweise die Mindestanzahl an Hydroxygruppen pro Molekül nicht erreicht werden, bevor die kinematische Viskosität über 8000 $mm^2/s$ steigt, so kann durch eine Veränderung insbesondere Verminderung der Reaktionstemperatur die Umsetzung so beeinflußt werden, daß ein Produkt mit den gewünschten Spezifikationen erhalten wird.

Geeignete Reaktionstemperaturen liegen beispielsweise im Bereich von 60-140°C, vorzugsweise 75-125°C.

Die Dauer der Umsetzung wird so gewählt, daß ein Produkt mit den geforderten Eigenschaften erhalten wird. Beispielsweise eignet sich für die Umsetzung ein Zeitraum von 20-60 Stunden.

Der Druck, bei dem die Umsetzung ausgeführt wird, spielt nur eine untergeordnete Rolle und kann vom Fachmann geeignet gewählt werden. Beispielsweise kann die Umsetzung bei einem Druck von 100-400 kPa durchgeführt werden.

Für Rapsöl hat sich eine Umsetzung mit Luft bei einer Temperatur von ca. 120°C für ca. 54 h als vorteilhaft erwiesen. Calendulaöl wird vorteilhaft mit Luft bei einer Temperatur von ca. 80°C für ca. 27 h umgesetzt. Diese Angaben sind jedoch nur als besonders bevorzugte Ausführungsformen zu verstehen. Der Fachmann wird erkennen, daß durch eine Variation der genannten Reaktionsbedingungen der Polymerisationsgrad und die Funktionalität des erhaltenen Produkts an eine gewünschte Verwendung beispielsweise als Monomerbaustein für die Kunststoffherstellung angepaßt werden kann.

Die vorliegende Erfindung umfaßt auch die nach dem vorstehend beschriebenen Verfahren erhältlichen Polyole sowie deren Verwendung zur Herstellung von Kunststoffen, insbesondere Polyurethanen.

Besondere Vorteile der erfindungsgemäßen Polyole sind die schadstofffreie Herstellung sowie die biologische Abbaubarkeit und gute Umweltverträglichkeit. Durch das große Molekulargewicht der Polyole ergeben sich Vorteile beim Emissionsverhalten sowohl im Rohzustand als auch in entsprechenden Rezepturen zur Herstellung von Kunststoffen.

Die nachfolgenden Beispiele sollen die Erfindung näher veranschaulichen, ohne jedoch die Erfindung hierauf zu beschränken.

Die folgenden Kenndaten wurden sowohl für die in den Beispielen eingesetzten Öle als auch für die erhaltenen Polyole bestimmt:

Die Iodzahl (IZ) ist ein Maß für die Menge an Mehrfachbindungen in einer Probe. Die Iodzahl wird in mg Iod/g Probe angegeben und definiert die Menge Iod, die von den Mehrfachbindungen der Probe verbraucht wird. Die Iodzahl wird nach ASTM D 1159-66 bestimmt.

Die Hydroxylzahl (OHZ) gibt Aufschluß über die Menge an Hydroxygruppen in einer Probe. Sie bezieht sich auf eine bestimmte Probenmenge, d.h. sie wird in mg KOH/g Probe angegeben. Eine OHZ von 56 entspricht daher einem Mol Hydroxygruppen pro kg Probe. Ist die Molmasse einer Probe bekannt, kann mit Hilfe der Hydroxylzahl die Funktionalität, d.h. die Anzahl der OH-Gruppe pro Molekül, bestimmt werden. Die Hydroxylzahl wird nach der Metrohm-Vorschrift 141/2 d bestimmt.

Die kinematische Viskosität (V40) der erhaltenen Produkte wird mittels eines Fallrohr- oder Steigrohrviskosimeters nach DIN 51562 bestimmt. Die Viskosität wird in allen Fällen bei 40°C gemessen und in $mm^2/s$ angegeben.

Die Neutralisationszahl (NZ) gibt den Gehalt an freien Fettsäuren in der Probe wieder. Hierdurch kann der Reinheitsgrad des eingesetzten Öls bestimmt werden. Außerdem kann nach Durchführung der Umsetzung überprüft werden, ob es zu Zersetzungsreaktionen gekommen ist. Die Neutralisationszahl wird mittels Farbindikator-Titration nach DIN 51558 Teil 1 bestimmt und in mg KOH/g Probe angegeben.

Folgende raffinierte Öle wurden in den Beispielen eingesetzt:

Tabelle 1

| Öl | IZ | OHZ | V40 | NZ |
|---|---|---|---|---|
| Calendula | 135 | 10,5 | 87,0 | 0,55 |
| Raps | 105 | 4 | 35,2 | 0,11 |

Beispiel 1

Für die Umsetzung wurden 700 ml Rapsöl in einen 1000 ml Dreihalskolben eingefüllt. Der Kolben befand sich in einem thermostatisch gesteuerten Ölbad, das über ein Thermometer in dem Kolben geregelt wurde. Luft wurde durch einen Gummischlauch mit einer Fritte als Abschluß unterhalb des Ölspiegels eingeblasen. Die Luft wurde zuvor zur Trocknung durch Silikaperlen geführt. Mittels eines Durchflußmeßgeräts wurde die Luftmenge auf 8 l/min eingeregelt. Die entweichende Luft wurde durch Isopopanol abgeleitet, um eventuell bei der Reaktion entstehendes Acrolein zu binden.

Die Temperatur wurde auf 120°C eingestellt und die Umsetzung erfolgte über sechs Tage bei einer Versuchsdauer von neun Stunden pro Tag, wobei das Ölbad über Nacht auf eine Temperatur von 50°C abgekühlt wurde und die Luftzufuhr unterbrochen wurde. Dies entspricht einer Umsetzungsdauer von 54 h.

Zu Beginn des Versuchs und am Morgen eines jeden folgenden Tages wurde eine 50 ml Probe entnommen und analysiert. Der Versuch wurde drei Mal wiederholt. Die Mittelwerte der Versuchsergebnisse sind in der folgenden Tabelle 2 zusammengefaßt.

Tabelle 2

| Probe | V40 | IZ | OHZ | NZ |
|---|---|---|---|---|
| Beginn | 35,0 | 105 | 4 | 0,11 |
| 1. Tag | 47 | 82 | 6 | |
| 2. Tag | 132 | 68 | 18 | |
| 3. Tag | 241 | 62 | 25 | |
| 4. Tag | 508 | 47 | 37 | |
| 5. Tag | 1000 | 43 | 49 | |
| 6. Tag | 1045 | 42 | 53 | 0,26 |

Man erkennt, daß die Viskosität (V40) der Probe während des Versuchs ansteigt und einen gewünschten Wert erreicht. Die Iodzahl (IZ) sinkt bis auf ein Minimum von ca. 40 mg KOH/g, sie kennzeichnet die fortschreitende Alterung des Öls. Die Hydroxylzahl (OHZ) erreicht ein Maximum von 53 mg KOH/g. Zusammen mit der relativen Molmasse des Produkts kann hieraus dessen Funktionalität errechnet werden. Alternativ kann durch eine Messung der Funktionalität die relative Molmasse des Produkts und damit der Oligomerisierungsgrad des Öls errechnet werden. Entsprechende Ergebnisse werden in Beispiel 3 gezeigt.

Die Neutralisationszahl (NZ) wurde nur zu Beginn und am Ende des Versuchs bestimmt. Man erkennt, daß keine wesentliche Zersetzung stattgefunden hat.

Beispiel 2

Beispiel 1 wurde mit Calendulaöl bei 80°C wiederholt. Der Versuch wurde nach drei Tagen beendet, da das Öl im Verlauf des vierten Tages die vorgesehene Viskositätsobergrenze überschritt. Dies entspricht einer Umsetzungsdauer von 27 h.

Der Versuch wurde einmal wiederholt und die Mittelwerte der Ergebnisse sind in der folgenden Tabelle 3 wiedergegeben.

Tabelle 3

| Probe | V40 | IZ | OHZ | NZ |
|---|---|---|---|---|
| Beginn | 87 | 136 | 11 | 0,55 |
| 1. Tag | 215 | 122 | 56 | |
| 2. Tag | 420 | 92 | 49 | |
| 3. Tag | 1900 | 76 | 38 | 0,80 |

Die Viskosität (V40) erreicht während des Versuchs den gewünschten Wert. Die sinkende Iodzahl (IZ) zeigt die Alterung des Öls an. Die Hydroxylzahl (OHZ) überschreitet am 1. Tag ein Maximum und sinkt dann ab, ein Zeichen für die fortschreitende Polymerisation der Probe. Die Neutralisationszahl (NZ) zu Beginn und am Ende der Umsetzung zeigt, daß keine wesentliche Zersetzung stattgefunden hat.

<u>Beispiel 3</u>

In diesem Beispiel wurden gemäß dem in den Beispielen 1 und 2 beschriebenen Verfahren verschiedene Polyolproben hergestellt. Das als Edukt eingesetzte Öl, die Umsetzungsbedingungen, die Viskosität (V40) und die Hydroxylzahl (OHZ) der erhaltenen Polyole sind in Tabelle 4 angegeben.

Tabelle 4 gibt darüber hinaus die experimentell bestimmte Funktionalität der erhaltenen Polyole sowie die daraus berechnete Molmasse (in g/mol) und die Anzahl n der Monomere (d.h. der Fettmoleküle des Ausgangsöls), die sich durchschnittlich zu einem Oligomer verbunden haben, an. Für beide Ausgangsöle wurde eine durchschnittliche Molmasse der Fettmoleküle von 280 g/mol angenommen.

Die Funktionalität der erhaltenen Polyole, d.h. die mittlere Anzahl an Hydroxygruppen pro Molekül, wurde durch Bestimmung des Gelpunkts experimentell ermittelt. Die Bestimmung der Funktionalität multifunktioneller Monomere mittels des Gelpunkts ist in der Literatur beschrieben (J. Ulbricht "Grundlagen der Synthese von Polymeren", Akademieverlag Berlin, 1978 und B. Tiedke "Makromolekulare Chemie", VCH Verlagsgesellschaft mbH, Weinheim, 1997).

Tabelle 4

| Ausgangsöl | Umsetzungsbedingungen | | V40 | OHZ | Funktionalität | Molmasse | n |
|---|---|---|---|---|---|---|---|
| | T/°C | Zeit/h | | | | | |
| Calendula | 80 | 27 | 1900 | 38 | 2,6 | 3838 | 14 |
| Calendula | 80 | 18 | 1270 | 44 | 2,4 | 3060 | 11 |
| Calendula | 70 | 20 | 1300 | 56 | 2,8 | 2805 | 10 |
| Raps | 120 | 54 | 1050 | 53 | 2,5 | 2646 | 10 |
| Raps | 140 | 45 | 1500 | 62 | 2,9 | 2624 | 9 |
| Raps | 140 | 54 | 2500 | 42 | 5,4 | 7213 | 26 |

Die vorstehenden Ergebnisse zeigen, daß mit dem erfindungsgemäßen Verfahren aus nativen Ölen Polyole mit einer Funktionalität (mittleren Anzahl an Hydroxygruppen pro Molekül) von mindestens zwei erhalten werden. Diese Polyole eignen sich als Monomerbausteine für die Kunststoffherstellung und insbesondere zur Umsetzung mit Di- oder Polyisocyanaten für die Polyurethanherstellung.

**Patentansprüche**

1. Verfahren zur Herstellung von Polyolen, **dadurch gekennzeichnet, daß** ein natives Öl so lange mit einem sauerstoffhaltigen Gas umgesetzt wird, bis das erhaltene Produkt eine kinematische Viskosität bei 40°C im Bereich von 100-8000 $mm^2/s$ und eine mittlere Anzahl an Hydroxygruppen pro Molekül von mindestens zwei aufweist.

2. Verfahren nach Anspruch 1, **dadurch gekennzeichnet, daß** das native Öl ungesättigte, vorzugsweise mehrfach ungesättigte Fettsäurereste umfaßt.

3. Verfahren nach einem der vorhergehenden Ansprüche, **dadurch gekennzeichnet, daß** das native Öl einen Mindestgehalt an ungesättigten Fettsäureresten von ca. 80% bezogen auf die Gesamtzahl der Fettsäurereste aufweist.

4. Verfahren nach Anspruch 1, **dadurch gekennzeichnet, daß** das native Öl ausgewählt ist aus der Gruppe bestehend aus Calendulaöl, Rapsöl und Sojaöl.

5. Verfahren nach einem der vorhergehenden Ansprüche, **dadurch gekennzeichnet, daß** das sauerstoffhaltige Gas Luft ist.

6. Verfahren nach einem der vorhergehenden Ansprüche, **dadurch gekennzeichnet, daß** das erhaltene Produkt eine kinematische Viskosität bei 40°C im Bereich von 400-5000 $mm^2/s$ vorzugsweise 1000-3000 $mm^2/s$ aufweist.

7. Verfahren nach einem der vorhergehenden Ansprüche, **dadurch gekennzeichnet, daß** die Umsetzung bei einer Temperatur von 60-140°C, vorzugsweise 75-125°C erfolgt.

8. Verfahren nach einem der vorhergehenden Ansprüche, **dadurch gekennzeichnet, daß** die Umsetzung für einen Zeitraum von 20-60 Stunden erfolgt.

9. Verfahren nach einem der vorhergehenden Ansprüche, **dadurch gekennzeichnet, daß** die Umsetzung bei einem Druck von 100-400 kPa durchgeführt wird.

10. Verfahren nach einem der vorhergehenden Ansprüche, **dadurch gekennzeichnet, daß** Rapsöl mit Luft bei einer Temperatur von ca. 120°C für ca. 54 Stunden umgesetzt wird.

11. Verfahren nach einem der Ansprüche 1-9, **dadurch gekennzeichnet, daß** Calendulaöl mit Luft bei einer Temperatur von ca. 80°C für ca. 27 Stunden umgesetzt wird.

12. Polyol, erhältlich nach dem Verfahren gemäß einem der Ansprüche 1-11.

13. Verwendung des Polyols nach Anspruch 12 zur Herstellung von Kunststoffen, insbesondere Polyurethanen.

Europäisches Patentamt

# EUROPÄISCHER RECHERCHENBERICHT

Nummer der Anmeldung
EP 00 11 3103

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | P. DAUTE, ET AL.: "Hydrolysestabile fettchemische Polyole für Polyurethananwendungen" FETT WISSENSCHAFT TECHNOLOGIE- FAT SCIENCE TECHNOLOGY., Bd. 95, Nr. 3, März 1993 (1993-03), Seiten 91-94, XP002154958 Konradin-Industrieverlag, Leinfelden-Echterdingen, DE ISSN: 0931-5985 * das ganze Dokument * | 1 | C07C29/00<br>C07C29/50<br>C07C51/367 |
| A | EP 0 295 536 A (HENKEL) 21. Dezember 1988 (1988-12-21) * das ganze Dokument * | 1 | |

RECHERCHIERTE SACHGEBIETE (Int.Cl.7)

C07C

Der vorliegende Recherchenbericht wurde für alle Patentansprüche erstellt

| Recherchenort | Abschlußdatum der Recherche | Prüfer |
|---|---|---|
| DEN HAAG | 8. Dezember 2000 | English, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.** EP 00 11 3103

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-12-2000

| Im Recherchenbericht angeführtes Patentdokument | Datum der Veröffentlichung | Mitglied(er) der Patentfamilie | Datum der Veröffentlichung |
|---|---|---|---|
| EP 0295536 A | 21-12-1988 | DE 3719790 A | 22-12-1988 |
| | | BR 8802843 A | 03-01-1989 |
| | | JP 1013045 A | 17-01-1989 |
| | | US 4825004 A | 25-04-1989 |

EPO FORM P0461

Für nähere Einzelheiten zu diesem Anhang : siehe Amtsblatt des Europäischen Patentamts, Nr.12/82